(19)

Europäisches
Patentamt
European
Patent Office
Office européen
des brevets

(11) **EP 3 848 398 A1**

(12) **EUROPEAN PATENT APPLICATION**
published in accordance with Art. 153(4) EPC

(43) Date of publication:
**14.07.2021 Bulletin 2021/28**

(21) Application number: **19857938.5**

(22) Date of filing: **21.08.2019**

(51) Int Cl.:
*C08B 15/04* (2006.01)      *A23L 29/262* (2016.01)
*A61K 8/73* (2006.01)      *D21H 11/20* (2006.01)

(86) International application number:
**PCT/JP2019/032536**

(87) International publication number:
**WO 2020/050015 (12.03.2020 Gazette 2020/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **04.09.2018 JP 2018165280**

(71) Applicant: **Nippon Paper Industries Co., Ltd.
Tokyo 114-0002 (JP)**

(72) Inventors:
• **MATSUMOTO Makoto**
  **Tokyo 114-0002 (JP)**
• **NAKAYAMA Takeshi**
  **Tokyo 114-0002 (JP)**
• **TAMURA Naoyuki**
  **Tokyo 114-0002 (JP)**

(74) Representative: **Kuhnen & Wacker
Patent- und Rechtsanwaltsbüro PartG mbB
Prinz-Ludwig-Straße 40A
85354 Freising (DE)**

(54) **OXIDIZED CELLULOSE NANOFIBERS AND DISPERSION OF OXIDIZED CELLULOSE NANOFIBERS**

(57)      Oxidized cellulose nanofibers characterized in that an aqueous dispersion of the oxidized cellulose nanofibers which has undergone concentration adjustment has a retention of a viscosity of 50% or higher measured immediately after 30-minute stirring with Disper at a rotation speed of 1,000 rpm, with respect to the viscosity of the aqueous dispersion of the oxidized cellulose nanofibers measured immediately after the concentration adjustment.

EP 3 848 398 A1

**Description**

Technical Field

The present invention relates to an oxidized cellulose nanofiber, and a dispersion containing the oxidized cellulose nanofiber.

Background Art

It is expected that various convenient new materials and devices are created from nanotechnology that is a technique for freely controlling substances in the region of nanometers, that is, on the scale of atoms and molecules. A cellulose nanofiber obtained by finely breaking a plant fiber is one of such new materials, and the cellulose nanofiber is characterized by having extremely high crystallinity, a low thermal expansion coefficient, and a high elastic modulus, and further has a high aspect ratio, so that the cellulose nanofiber is expected to have effects such as imparting strength and stabilizing shape by being made into a composite of rubber or resin. Further, this cellulose nanofiber has viscosity characteristics such as pseudoplasticity and thixotropy in a state of dispersion, and is expected to be effective as an additive agent such as a thickener.

Various developments and studies have been conducted on this cellulose nanofiber, and for example, Patent Literature 1 discloses a fine cellulose fiber (cellulose nanofiber) having a number average fiber diameter of 2 to 150 nm, in which a part of hydroxyl groups of the cellulose is carboxylated. This cellulose nanofiber has a characteristic that the viscosity at a low shear rate is high and the viscosity at a high shear rate is low. Since the cellulose nanofiber has such a viscosity characteristic, the cellulose nanofiber is used as a thickener in various fields of cosmetics, food, civil engineering, building material, ink, coating material and the like.

Citation List

Patent Literature

Patent Literature 1: JP 2008-1728 A

Summary of Invention

Technical Problem

However, in a case where the cellulose nanofiber disclosed in Patent Literature 1 is use as a thickener, there has been a problem that the viscosity is significantly lowered when stirring treatment is performed in mixing the cellulose nanofiber with a partner to which the thickening function is imparted.

For this reason, it is an object of the present invention to provide an oxidized cellulose nanofiber having an excellent viscosity retention even when the stirring treatment is performed in mixing the cellulose nanofiber with a partner to which the thickening function is imparted. Further, an object of the present invention to provide a dispersion containing the oxidized cellulose nanofiber.

Solution to Problem

The present invention provides the following (1) to (7) .

(1) An oxidized cellulose nanofiber, satisfying a following condition: an aqueous dispersion of the oxidized cellulose nanofiber which has undergone concentration adjustment has a retention of a viscosity of 50% or higher measured immediately after 30-minute stirring with Disper at a rotation speed of 1,000 rpm, with respect to the viscosity of the aqueous dispersion of the oxidized cellulose nanofiber measured immediately after the concentration adjustment.
(2) The oxidized cellulose nanofiber described in (1), satisfying a following condition: a 0.7 mass% adjusted aqueous dispersion of the oxidized cellulose nanofiber which has undergone concentration adjustment has a retention of a Brookfield viscosity (at a rotation speed of 6 rpm) of 50% or higher measured immediately after 30-minute stirring with Disper at a rotation speed of 1,000 rpm, with respect to the Brookfield viscosity (at a rotation speed of 6 rpm) of the 0.7 mass% adjusted aqueous dispersion of the oxidized cellulose nanofiber measured immediately after the concentration adjustment.
(3) The oxidized cellulose nanofiber described in (1) or (2), in which an amount of carboxy groups of the oxidized cellulose nanofiber is 0.4 to 1.0 mmol/g with respect to a bone dry mass of the oxidized cellulose nanofiber.

(4) The oxidized cellulose nanofiber described in (3), in which transparency in a 1.0 mass% aqueous dispersion of the oxidized cellulose nanofiber is 80% or more.

(5) The oxidized cellulose nanofiber described in (1) or (2), in which an amount of carboxy groups of the oxidized cellulose nanofiber is 0.2 to 2.0 mmol/g with respect to a bone dry mass of the oxidized cellulose nanofiber, and further a proportion of the carboxy groups in the oxidized cellulose nanofiber, determined by the following equation (1):

$$\text{Proportion of carboxy groups (\%)} = (\text{amount of carboxy}$$

$$\text{groups/total amount of carboxy groups and carboxylate}$$

$$\text{groups}) \times 100,$$

is 50% or more.

(6) The oxidized cellulose nanofiber described in (1) or (2), in which an amount of carboxy groups of the oxidized cellulose nanofiber is 0.2 to 2.0 mmol/g with respect to a bone dry mass of the oxidized cellulose nanofiber, and a polyvalent metal is contained in an amount of 40 to 100 mol% with respect to the amount of the carboxy groups of the oxidized cellulose nanofiber.

(7) A dispersion of oxidized cellulose nanofiber, including the oxidized cellulose nanofiber described in any one of claims (1) to (6).

Advantageous Effects of Invention

According to the present invention, an oxidized cellulose nanofiber having an excellent viscosity retention even when the stirring treatment is performed in mixing the cellulose nanofiber with a partner to which the thickening function is imparted can be provided. Further, a dispersion containing the oxidized cellulose nanofiber can be provided.

Description of Embodiments

The oxidized cellulose nanofiber (oxidized CNF) according to the present invention is characterized by satisfying the following condition.

Condition: the retention of viscosity of an aqueous dispersion of oxidized cellulose nanofiber which has undergone concentration adjustment, measured immediately after 30-minute stirring with Disper at a rotation speed of 1,000 rpm is 50% or higher, with respect to the viscosity of the aqueous dispersion of oxidized cellulose nanofiber measured immediately after the concentration adjustment.

In this regard, it is preferable that the oxidized cellulose nanofiber (oxidized CNF) according to the present invention satisfies the following condition.

Condition: the retention of Brookfield viscosity (at a rotation speed of 6 rpm) of a 0.7 mass% adjusted aqueous dispersion of oxidized cellulose nanofiber which has undergone concentration adjustment, measured immediately after 30-minute stirring with Disper at a rotation speed of 1,000 rpm of is 50% or higher, with respect to the Brookfield viscosity (at a rotation speed of 6 rpm) of the 0.7 mass% adjusted aqueous dispersion of oxidized cellulose nanofiber measured immediately after the concentration adjustment.

The oxidized cellulose nanofiber according to the present invention is not particularly limited as long as it satisfies the above viscosity retention, but examples of the oxidized cellulose nanofiber include: 1) an oxidized CNF in which the amount of carboxy groups is 0.4 to 1.0 mmol/g with respect to the bone dry mass of the oxidized CNF; 2) an oxidized CNF in which the amount of carboxy groups is 0.4 to 1.0 mmol/g with respect to the bone dry mass of the oxidized CNF, and of which the transparency in a 1.0 mass% aqueous dispersion is 80% or more; and 3) an oxidized CNF in which the amount of carboxy groups is 0.2 to 2.0 mmol/g with respect to the bone dry mass of the oxidized CNF, and further in which the proportion of carboxy groups obtained by the following equation (1) is 50% or more.

$$\text{Equation (1): proportion of carboxy groups (\%)} =$$

$$(\text{amount of carboxy groups/total amount of carboxy groups and}$$

$$\text{carboxylate groups}) \times 100$$

Note that, in the present specification, in a case where the proportion of carboxy groups is determined, the carboxy group is distinguished from the carboxylate group. Specifically, the carboxy group indicates a group represented by -COOH, and the carboxylate group indicates a group represented by -COO⁻. The counter cation of the carboxylate group is not particularly limited, and examples of the counter cation include alkali metal ions such as a sodium ion, and a potassium ion.

In addition, the oxidized cellulose nanofiber according to the present invention is not particularly limited as long as it satisfies the above viscosity retention, but an example of the oxidized cellulose nanofiber includes an oxidized CNF in which the amount of carboxy groups is 0.2 to 2.0 mmol/g with respect to the bone dry mass of the oxidized CNF, and further a polyvalent metal is contained in an amount of 40 to 100 mol% with respect to the amount of carboxy groups of the oxidized CNF.

(Oxidized CNF)

The oxidized CNF according to the present invention can be obtained by disintegrating the oxidized cellulose that is obtained by introducing a carboxy group into a cellulose raw material.

(Raw material)

Examples of the cellulose raw material include a plant material (for example, wood, bamboo, hemp, jute, kenaf, farm wastes, cloth, pulp (softwood unbleached kraft pulp (NUKP), softwood bleached kraft pulp (NBKP), hardwood unbleached kraft pulp (LUKP), hardwood bleached kraft pulp (LBKP), softwood unbleached sulfite pulp (NUSP), softwood bleached sulfite pulp (NBSP), thermomechanical pulp (TMP), regenerated pulp, or used paper), an animal material (for example, ascidians), algae, microorganisms (for example, acetic acid bacteria (Acetobacter)), and ones originating from a microbial product, and any one of the materials can be used. The cellulose raw material is preferably a cellulose raw material derived from a plant or a microorganism, and more preferably a plant-derived cellulose raw material.

(Introduction of carboxy group)

By oxidizing (carboxylating) the above-described cellulose raw material by a known method, a carboxy group can be introduced into the cellulose raw material.

As an example of the oxidation, there is a method of oxidizing a cellulose raw material in water by using an oxidizing agent in the presence of an N-oxyl compound and a bromide, iodide, or mixture of a bromide and an iodide. By this oxidation reaction, a primary hydroxyl group at the C6-position of a pyranose ring on a surface of the cellulose is selectively oxidized. As a result, oxidized cellulose having an aldehyde group and a carboxy group (-COOH) or a carboxylate group (-COO⁻) on the surface can be obtained. The concentration of cellulose during the reaction is not particularly limited, but is preferably 5% by mass or less.

The N-oxyl compound means a compound capable of generating a nitroxy radical. As the N-oxyl compound, any compound can be used as long as it is a compound that promotes the target oxidation reaction. Examples of the N-oxyl compound include 2,2,6,6-tetramethylpiperidine-1-oxy radical (TEMPO), and a derivative thereof (for example, 4-hydroxy TEMPO).

The amount of the N-oxyl compound to be used is not particularly limited as long as it is an amount of a catalyst capable of oxidizing the cellulose to be a raw material. The amount of the N-oxyl compound is preferably 0.01 mmol to 10 mmol, more preferably 0.01 mmol to 1 mmol, and furthermore preferably 0.05 mmol to 0.5 mmol, per gram of bone-dry cellulose. Further, the concentration is preferably around 0.1 mmol/L to 4 mmol/L with respect to the reaction system.

The bromide is a compound containing bromine, and includes an alkali metal bromide that can be dissociated and ionized in water. In addition, the iodide means a compound containing iodine, and includes an alkali metal iodide.

The amount of the bromide or iodide to be used can be selected in the range in which the oxidation reaction can be promoted. The total amount of the bromide and the iodide is preferably 0.1 mmol to 100 mmol, more preferably 0.1 mmol to 10 mmol, and furthermore preferably 0.5 mmol to 5 mmol, per gram of bone-dry cellulose.

As the oxidizing agent, a known one can be used, for example, halogen, hypohalous acid, halous acid, perhalogen acid, a salt of halogen, hypohalous acid, halous acid, or perhalogen acid, a halogen oxide, a peroxide, or the like can be used. Among them, sodium hypochlorite, which is inexpensive and has low environmental load, is preferable.

The amount of the oxidizing agent to be used is preferably 0.5 mmol to 500 mmol, more preferably 0.5 mmol to 50 mmol, and furthermore preferably 1 mmol to 25 mmol, per gram of bone-dry cellulose. Further, for example, the amount of the oxidizing agent is preferably 1 mol to 40 mol per mole of the N-oxyl compound.

In an oxidation step of cellulose, the reaction can proceed efficiently even under relatively mild conditions. For this reason, the reaction temperature is preferably 4°C to 40°C, and may be a room temperature of around 15°C to 30°C. As the reaction proceeds, a carboxy group is generated in cellulose, so that the pH of the reaction mixture is lowered.

In order to allow the oxidation reaction to proceed efficiently, it is preferable to add an alkaline solution such as an aqueous sodium hydroxide solution in the course of the reaction to maintain the pH of the reaction mixture at around 8 to 12, and preferably around 10 to 11. As the reaction medium, water is preferable because it is easy to handle and is less likely to cause a side reaction.

The reaction time in the oxidation reaction can be appropriately set in accordance with the degree of progress of the oxidation, and is usually 0.5 to 6 hours, and preferably 0.5 to 4 hours.

Further, the oxidation reaction may be performed by being divided into two stages. For example, by oxidizing again carboxylated cellulose, which has obtained by filtration after completion of the first stage, under the same or different reaction conditions, the oxidation can proceed efficiently without having any reaction inhibition due to the salt by-produced in the reaction of the first stage.

As another example, there is a method of performing the oxidation by bringing a gas containing ozone into contact with a cellulose raw material. By this oxidation reaction, hydroxyl groups at least at the 2- and 6-positions of a pyranose ring are oxidized, and further decomposition of the cellulose chain occurs.

The ozone concentration in the gas containing ozone is preferably 50 $g/m^3$ to 250 $g/m^3$, and more preferably 50 $g/m^3$ to 220 $g/m^3$. The amount of the ozone to be added to a cellulose raw material is preferably 0.1 parts by mass to 30 parts by mass, and more preferably 5 parts by mass to 30 parts by mass, when the solid content of the cellulose raw material is set to 100 parts by mass.

The temperature in ozone treatment is preferably 0°C to 50°C, and more preferably 20°C to 50°C. The time in the ozone treatment is not particularly limited, but is around 1 to 360 minutes, and preferably around 30 to 360 minutes. If the conditions of the ozone treatment are within these ranges, the cellulose can be prevented from being excessively oxidized and decomposed, and the yield of the oxidized cellulose becomes favorable.

After the ozone treatment, the additional oxidation treatment may be performed by using an oxidizing agent. The oxidizing agent to be used in the additional oxidation treatment is not particularly limited, and examples of the oxidizing agent include a chlorine-based compound such as chlorine dioxide, and sodium chlorite, oxygen, hydrogen peroxide, persulfuric acid, and peracetic acid. For example, the additional oxidation treatment can be performed by dissolving such an oxidizing agent in water or a polar organic solvent such as alcohol to prepare an oxidizing agent solution, and by immersing a cellulose raw material in the solution.

The amount of the carboxy groups, which indicates the degree of modification of oxidized cellulose can be adjusted by controlling the amount of the above oxidizing agent to be added, and the reaction condition such as reaction time. In this regard, the amount of carboxy groups is around 0.2 to 2.0 mmol/g with respect to the bone dry mass of the oxidized cellulose. If the amount of carboxy groups is less than 0.2 mmol/g, a large amount of energy is required to disintegrate the oxidized cellulose into oxidized CNF. Further, in a case where oxidized cellulose in which the amount of carboxy groups exceeds 2.0 mmol is used as a raw material, the oxidized CNF to be obtained does not have a fiber form. Note that, in the present specification, in a case of indicating the degree of modification, the amount of carboxy groups indicates the total amount of carboxy groups (-COOH) and carboxylate groups (-COO⁻).

(Disintegration)

The device to be used for the disintegration is not particularly limited, and examples of the device include devices of high-speed rotary type, colloid mill type, high-pressure type, roll mill type, ultrasonic type, and the like, and a high-pressure or ultra high-pressure homogenizer is preferable, and a wet high-pressure or ultra high-pressure homogenizer is more preferable. The device is preferably capable of applying a strong shear force to a cellulose raw material or oxidized cellulose (usually a dispersion). The pressure that can be applied by the device is preferably 50 MPa or more, more preferably 100 MPa or more, and furthermore preferably 140 MPa or more. As the device, a wet high-pressure or ultra high-pressure homogenizer that can apply the above pressure to a cellulose raw material or oxidized cellulose (usually a dispersion), and further can apply a strong shear force is preferable. With this device, the disintegration can be efficiently performed. The number of treatments (passes) by the disintegration device may be once, or twice or more, and is preferably twice or more.

In the dispersion treatment, in general, oxidized cellulose is dispersed in a solvent. The solvent is not particularly limited as long as it can disperse the oxidized cellulose, but examples of the solvent include water, an organic solvent (for example, a hydrophilic organic solvent such as methanol), and a mixed solvent of water and an organic solvent. Since the cellulose raw material is hydrophilic, as the solvent, water is preferable.

The solid content concentration of the oxidized cellulose in the dispersion is usually 0.1% by mass or more, preferably 0.2% by mass or more, and more preferably 0.3% by mass or more. With this arrangement, the amount of liquid with respect to the amount of cellulose fiber raw material becomes appropriate, and is efficient. The upper limit is usually 10% by mass or less, and preferably 6% by mass or less. With this arrangement, the flowability can be retained.

The order of the disintegration treatment and the dispersion treatment is not particularly limited, and either of them may be performed first, or both of them may be performed at the same time, but it is preferable to perform the dispersion treatment first, and then the disintegration treatment. The combination of respective treatments may be performed at least once, or may be repeated twice or more.

Pretreatment may be performed as needed prior to the disintegration treatment or the dispersion treatment. The pretreatment may be performed by using a mixing, stirring, emulsifying and dispersing device such as a high-speed shear mixer.

The average fiber diameter of the oxidized CNF according to the present invention is preferably 3 nm or more or 500 nm or less. In the measurement of the average fiber diameter and average fiber length of cellulose nanofibers, for example, by preparing a 0.001 mass% aqueous dispersion of oxidized CNF, spreading thinly the diluted dispersion on a mica sample table, heat drying the dispersion at 50°C to prepare a sample for observation, and measuring the height of the cross section of the shape image observed with an atomic force microscope (AFM), the number average fiber diameter or fiber length can be calculated.

Further, the average aspect ratio of oxidized CNF is usually 50 or more. The upper limit is not particularly limited, but is usually 1000 or less. The average aspect ratio can be calculated by the following equation:

$$\text{Aspect ratio} = \text{average fiber length} / \text{average fiber diameter}$$

(Transparency)

In the present specification, the transparency refers to the transmittance of light having a wavelength of 660 nm when the oxidized CNF is used as an aqueous dispersion having a solid content of 1% (w/v). The method for measuring the transparency of oxidized CNF is as follows:

A CNF dispersion (solid content: 1% (w/v), and dispersion medium: water) is prepared, and the transmittance of light having a wavelength of 660 nm is measured by using a UV-VIS spectrophotometer, UV-1800 (manufactured by Shimadzu Corporation), with a square cell having an optical path length of 10 mm.

The transparency of the oxidized CNF according to the present invention is 60% or more. The transparency is more preferably 60 to 100%, furthermore preferably 70 to 100%, and still more preferably 80 to 100%. When the amount of carboxy groups of such oxidized CNF is 0.4 to 1.0 mmol/g with respect to the bone dry mass of the oxidized CNF, an oxidized cellulose nanofiber of which the viscosity reduction is suppressed in a low shear rate range can be provided.

If the amount of carboxy groups of the oxidized CNF is a high value of, for example, 1.2 to 2.0 mmol/g, the effect of charge repulsion due to the carboxy group on a surface of the oxidized CNF is large, and the fiber is easily affected by shearing when the aqueous dispersion of the oxidized CNF is subjected to stirring treatment, so that it is considered that the retention of the viscosity is difficult. On the other hand, if the amount of carboxy groups of the oxidized CNF is a value as low as 0.4 to 1.0 mmol/g, the charge on a surface of the oxidized CNF is reduced, so that it is presumed that the effect of the repulsion is weakened and the interaction between the oxidized CNFs becomes easier to work, and thus the viscosity when the fibers are subjected to shearing can be retained.

In general, in a case where the oxidized CNF is made to have a high transparency of 60% or more, it is known that by setting the amount of carboxy groups of the oxidized CNF to a high value of, for example, 1.2 to 2.0 mmol/g, the above-described disintegration treatment is facilitated, and as a result of which oxidized CNF with high transparency can be easily obtained. On the other hand, in the present patent application, a new effect that by performing sufficiently the disintegration treatment for making the oxidized CNF to have a transparency of 80% or more at a value as low as 0.4 to 1.0 mmol/g of the amount of carboxy groups of the oxidized CNF, the viscosity retention in a low shear rate range is improved has been found.

The reason why the effect described above can be obtained is not necessarily clear, but it is presumed that by disintegrating sufficiently modified pulp with a low degree of modification, a part having a hydroxyl group, of which the surface has not been chemically treated, is exposed, the oxidized CNFs easily form a hydrogen bond with each other in addition to the reduction in the surface charge of the oxidized CNFs, and thus the viscosity at low shearing is retained.

(Specific Example 1 of the oxidized CNF according to the present invention)

As the oxidized CNF having a retention of a Brookfield viscosity (at a rotation speed of 6 rpm) of 50% or higher, measured immediately after 30-minute stirring of a 0.7 mass% adjusted aqueous dispersion of the oxidized CNF, which has undergone concentration adjustment, with Disper at a rotation speed of 1,000 rpm, with respect to the Brookfield viscosity (at a rotation speed of 6 rpm) of the 0.7 mass% adjusted aqueous dispersion of the oxidized CNF measured immediately after the concentration adjustment, an oxidized CNF in which the amount of carboxy groups is 0.4 to 1.0 mmol/g with respect to the bone dry mass of the oxidized CNF can be mentioned. In the above-described method for producing oxidized cellulose, the oxidized CNF in which the amount of carboxy groups is 0.4 to 1.0 mmol/g can be prepared by controlling the amount of an oxidizing agent to be added, and the reaction condition such as reaction time.

(Specific Example 2 of the oxidized CNF according to the present invention)

As the oxidized CNF having a retention of a Brookfield viscosity (at a rotation speed of 6 rpm) of 50% or higher, measured immediately after 30-minute stirring of a 0.7 mass% adjusted aqueous dispersion of the oxidized CNF, which has undergone concentration adjustment, with Disper at a rotation speed of 1,000 rpm, with respect to the Brookfield viscosity (at a rotation speed of 6 rpm) of the 0.7 mass% adjusted aqueous dispersion of the oxidized CNF measured immediately after the concentration adjustment, an oxidized CNF in which the amount of carboxy groups is 0.4 to 1.0 mmol/g with respect to the bone dry mass of the oxidized CNF, and further of which the transparency in a 1.0 mass% aqueous dispersion is 80% or more can be mentioned. In the above-described method for producing oxidized cellulose, the oxidized CNF in which the amount of carboxy groups is 0.4 to 1.0 mmol/g can be prepared by controlling the amount of an oxidizing agent to be added, and the reaction condition such as reaction time.

(Specific Example 3 of the oxidized CNF according to the present invention)

As the oxidized CNF having a retention of a Brookfield viscosity (at a rotation speed of 6 rpm) of 50% or higher, measured immediately after 30-minute stirring of a 0.7 mass% adjusted aqueous dispersion of the oxidized CNF, which has undergone concentration adjustment, with Disper at a rotation speed of 1,000 rpm, with respect to the Brookfield viscosity (at a rotation speed of 6 rpm) of the 0.7 mass% adjusted aqueous dispersion of the oxidized CNF measured immediately after the concentration adjustment, an oxidized CNF in which the amount of carboxy groups is 0.2 to 2.0 mmol/g, and further in which the proportion of carboxy groups determined by the following equation (1) is 50% or more can be mentioned.

$$\text{Equation (1): proportion of carboxy groups (\%) =}$$
$$\text{(amount of carboxy groups/total amount of carboxy groups and}$$
$$\text{carboxylate groups)} \times 100$$

The proportion of the carboxy groups in the oxidized CNF, determined by the above equation (1) can be adjusted by desalting the oxidized CNF with a cation exchange resin or by the addition of an acid.

(Desalting treatment)

By bringing the oxidized CNF into contact with a cation exchange resin, a cation salt (Na salt) is replaced with a proton. Since a cation exchange resin is used, an unnecessary by-product such as sodium chloride is not generated, and after the acid treatment using the cation exchange resin, only by filtering and removing the cation exchange resin with a metal mesh or the like, an aqueous dispersion of acid-type/salt-type adjusted oxidized CNF can be obtained as a filtrate.

The target to be removed as the residue with a metal mesh or the like is the cation exchange resin, and the oxidized CNF is hardly removed with a metal mesh or the like having such a diameter as the metal mesh, and almost the whole amount is contained in the filtrate. In the filtrate, an oxidized CNF having an extremely short fiber length is contained in a large amount. Further, since the filtrate does not need to be washed or dehydrated, the oxidized CNF is hardly aggregated. Therefore, an aqueous dispersion of oxidized CNF having high light transmittance can be obtained.

As the cation exchange resin, a strong acid ion-exchange resin or a weak acid ion-exchange resin can be used as long as the counter ion is a proton. Among them, a strong acid ion-exchange resin is preferably used. Examples of the strong acid ion-exchange resin and weak acid ion-exchange resin include ones obtained by introducing a sulfonic acid group or a carboxy group into a styrene-based resin or an acrylic resin.

As the shape of the cation exchange resin, it is not particularly limited, and ones in various shape of a fine grain (granular shape), a film, a fiber and the like can be used. Among them, a granular shape is preferable from the viewpoint of efficiently treating the oxidized CNF and facilitating separation after the treatment. As such a cation exchange resin, a commercially available product can be used. Examples of the commercially available product include Amberjet 1020, Amberjet 1024, Amberjet 1060, and Amberjet 1220 (all manufactured by ORGANO CORPORATION), Amberlite IR-200C, and Amberlite IR-120B (all manufactured by Tokyo Organic Chemical Ind., Ltd.), Lewatit SP 112, and Lewatit S100 (all manufactured by Bayer AG), GEL CK08P (manufactured by Mitsubishi Chemical Corporation), and Dowex 50W-X8 (manufactured by The Dow Chemical Company).

The contact between the oxidized CNF and the cation exchange resin can be performed, for example, by mixing a cation exchange resin having a granular shape with an aqueous dispersion of oxidized CNF to allow the oxidized CNF to come into contact with the cation exchange resin for a certain period of time while stirring and shaking the mixture as needed, and then by separating the cation exchange resin from the aqueous dispersion.

The concentration of the aqueous dispersion of oxidized CNF, and the ratio with the cation exchange resin are not particularly limited, and can be appropriately set by a person skilled in the art from the viewpoint of efficiently performing the proton replacement. As an example, the concentration of the aqueous dispersion of oxidized CNF is preferably 0.05 to 10% by mass. If the concentration of the aqueous dispersion is less than 0.05% by mass, the time required for the proton replacement may be extremely long. If the concentration of the aqueous dispersion is exceeding 10% by mass, the sufficient effect of the proton replacement may not be obtained. The contact time is not particularly limited, and can be appropriately set by a person skilled in the art from the viewpoint of efficiently performing the proton replacement. For example, the proton replacement can be performed by the contact for 0.25 to 4 hours.

In this case, the oxidized CNF is allowed to come into contact with the cation exchange resin for a sufficient period of time by using an appropriate amount of cation exchange resin, and then by removing the cation exchange resin with a metal mesh or the like as the residue, an acid-type (hereinafter, may also be referred to as "H-type") oxidized CNF can be produced.

(Acid addition treatment)

The acid addition treatment is a treatment for adding an acid into a dispersion of oxidized CNF. The acid may be an inorganic acid or an organic acid. Examples of the inorganic acid include mineral acids such as sulfuric acid, hydrochloric acid, nitric acid, sulfurous acid, nitrous acid, phosphoric acid, and residual acid of a chlorine dioxide generator, and the inorganic acid is suitably hydrochloric acid. Examples of the organic acid include acetic acid, lactic acid, oxalic acid, citric acid, and formic acid. The pH at the time of acid treatment is usually 2 or more, and preferably 3 or more. The upper limit is preferably 6 or less, and preferably 5 or less. Therefore, the pH is preferably 2 to 6, more preferably 2 to 5, and furthermore preferably 3 to 5. The amount of the acid to be added is not particularly limited, and the addition of the acid may be terminated at the time point when the oxidized CNFs aggregate and the translucent gelled substance precipitates.

It is preferable to perform cleaning treatment after the addition of the acid. In this way, a dispersion of oxidized CNF can be obtained.

It is preferable to perform the cleaning treatment to the extent that the residual salt is removed from the gelled substance. In this way, the storage stability and dispersibility of the oxidized CNF can be improved. The amount of the residual salt contained in the cleaning liquid after completion of the cleaning is not particularly limited, but is preferably 0.05% by mass or less, and more preferably the detection limit or less.

The method for the cleaning treatment is not particularly limited, and examples of the method include a method of preliminarily dehydrating, and washing a gelled substance obtained after acidification as needed, and then of dispersing and pulverizing the gelled substance, and a method of repeating a series of the processes twice or more. The solvent can be freely used as long as the oxidized CNF can be sufficiently dispersed in the solvent, and examples of the solvent include water, an organic solvent, and a mixed solvent of two or more solvents selected from water and organic solvents. The organic solvent is preferably a hydrophilic solvent such as methanol. The mixed solvent preferably contains at least water. Since the cellulose raw material is hydrophilic, the solvent is preferably water, a hydrophilic organic solvent, or a hydrophilic mixed solvent, and more preferably water. The amount of the solvent to be added is usually an amount with which the gelled substance has a solid content of around 1 to 2%.

The dispersion may be performed by using a slurrying device such as a mixer. It is preferable that the dispersion is performed until the gelled substance is made finer (slurried), that is, until the particle diameter reaches around a particle diameter with which the particles are not precipitated in a short time.

The pulverization is usually performed by using a pulverizer. Examples of the pulverizer include a pulverizer that uses a medium such as a bead mill, and a pulverizer that does not use any medium, such as a high-speed rotary type, a colloid mill type, a high-pressure type, a roll mill type, or an ultrasonic disperser. Among them, a pulverizer that does not use any medium is preferable, a high-pressure type disperser is more preferable, a wet high-pressure type disperser is furthermore preferable, and a wet high-pressure or ultra high-pressure homogenizer is still more preferable. In this way, a dispersion in which acid-type cellulose nanofibers are sufficiently dispersed can be efficiently obtained. The pulverization condition is preferably 50 MPa or more, more preferably 100 MPa or more, and furthermore preferably 140 MPa or more. It is preferable that the pulverizer has an ability to perform the dispersion under such a condition. The number of treatments (passes) by the pulverizer may be once, or twice or more.

In the cleaning treatment, dehydration may be performed as needed. An example of the dehydration includes dehydration by a centrifugal separation method. It is preferable that the dehydration is performed until the solid content in the solvent reaches around 3 to 20%.

The temperature of the cleaning treatment is preferably 10°C or more, and more preferably 20°C or more. The upper limit is preferably 50°C or less, and more preferably 40°C or less. Therefore, the temperature of the cleaning treatment is preferably 10 to 50°C, and more preferably 20 to 40°C.

In addition, the proportion of carboxy groups can be calculated by the following method.

First, 250 mL of a 0.1 mass% slurry of an oxidized (carboxylated) cellulose nanofiber salt is prepared. A 0.1 M aqueous hydrochloric acid solution is added to the prepared slurry to adjust the pH to 2.5, and then measurement of the electrical conductivity of the pH-adjusted mixture above is continued until the pH reaches 11 while adding a 0.1 N aqueous sodium hydroxide solution to the mixture. From the amount of the sodium hydroxide (a) consumed in the neutralization stage with a weak acid in which the change in the electrical conductivity is slow, the total amount of carboxy groups and carboxylate groups is calculated by using the following equation (2).

$$\text{Equation (2): total amount of carboxy groups and}$$
$$\text{carboxylate groups (mmol/g oxidized cellulose nanofiber}$$
$$\text{salt)} = a \text{ (mL)} \times 0.1/\text{mass (g) of oxidized cellulose}$$
$$\text{nanofiber salt}$$

Next, 250 mL of a 0.1 mass% slurry of desalted acid-type oxidized cellulose nanofibers is prepared. Measurement of the electrical conductivity of the slurry prepared above is continued until the pH reaches 11 while adding a 0.1 N aqueous sodium hydroxide solution to the slurry. From the amount of the sodium hydroxide (b) consumed in the neutralization stage with a weak acid in which the change in the electrical conductivity is slow, the amount of carboxy groups is calculated by using the following equation (3).

$$\text{Equation (3): amount of carboxy groups (mmol/g oxidized}$$
$$\text{cellulose nanofibers = b (mL)} \times 0.1/\text{mass (g) of oxidized}$$
$$\text{cellulose nanofiber}$$

From the calculated amount of carboxy groups and the total amount of carboxy groups and carboxylate groups, the proportion of carboxy groups can be calculated by using the following equation (1).

$$\text{Equation (1): proportion of carboxy groups (\%)} =$$
$$\text{(amount of carboxy groups/total amount of carboxy groups and}$$
$$\text{carboxylate groups)} \times 100$$

(Specific Example 4 of the oxidized CNF according to the present invention)

As the oxidized CNF having a retention of a Brookfield viscosity (at a rotation speed of 6 rpm) of 50% or higher, measured immediately after 30-minute stirring of a 0.7 mass% adjusted aqueous dispersion of the oxidized CNF, which has undergone concentration adjustment, with Disper at a rotation speed of 1,000 rpm, with respect to the Brookfield viscosity (at a rotation speed of 6 rpm) of the 0.7 mass% adjusted aqueous dispersion of the oxidized CNF measured immediately after the concentration adjustment, an oxidized CNF in which the amount of carboxy groups is 0.2 to 2.0 mmol/g, and further which contains a polyvalent metal in an amount of 40 to 100 mol% with respect to the amount of carboxy groups of the oxidized CNF, can be mentioned.

As the method for producing the dispersion of the oxidized CNF containing a polyvalent metal according to the present invention, for example, the following method can be mentioned.

First, an aqueous dispersion of oxidized CNF in which the amount of carboxy groups is adjusted to 0.2 to 2.0 mmol/g is prepared. Next, a polyvalent metal in an amount of 40 to 100 mol% with respect to the amount of carboxy groups contained in the oxidized CNF is added to and mixed with the aqueous dispersion of oxidized CNF. By passing through these steps, a dispersion of oxidized cellulose nanofibers, which contains the polyvalent metal in an amount of 40 to 100 mol% with respect to the amount of carboxy groups of the oxidized CNF, can be obtained.

When a crosslink by ionic bonding is formed between the carboxy groups of the oxidized CNF with the addition of the polyvalent metal, the network between cellulose nanofibers is strengthened, and the network structure can be maintained against shearing, so that the viscosity can be retained.

When the polyvalent metal is added to the dispersion of the oxidized CNF, the concentration of the dispersion of the oxidized CNF, the temperature, the pressure, and the like are not particularly limited, but it is preferable that the concentration of the dispersion is usually 0.5 to 1.2% by mass, and the temperature and the pressure are usually 18 to 35°C, and normal pressure, respectively.

Further, when the polyvalent metal is added to and mixed with the dispersion of the oxidized CNF, the rotation speed, the mixing time, and the like are not particularly limited as long as the polyvalent metal can be mixed homogeneously in the dispersion of the oxidized CNF, but it is preferable that the rotation speed is set to 1000 to 3000 rpm, and the mixing time is set to 5 to 30 minutes.

The polyvalent metal to be added to the above oxidized CNF is not particularly limited, but it is preferable to use a hydroxide, carbonate, or organic acid salt of a divalent or trivalent metal, more preferable to use a hydroxide, carbonate, or organic acid salt of calcium, zinc, aluminum, cobalt, nickel, or copper, furthermore preferable to use a hydroxide, carbonate, or organic acid salt of calcium, or aluminum, and particularly preferable to use a hydroxide, carbonate, or organic acid salt of calcium. In this regard, an example of the organic acid salt of calcium includes calcium acetate.

Further, the content of the polyvalent metal in the dispersion of the oxidized CNF can be confirmed qualitatively and quantitatively from ICP emission spectroscopic analysis, and elemental analysis such as fluorescent X-ray.

Examples

Hereinafter, the present invention will be described in more detail with reference to Examples, however, the present invention is not limited to the following Examples.

<Stability Test 1>

210 g of a 1.0 mass% aqueous dispersion of oxidized cellulose nanofibers was weighed and put in a 600-mL plastic container, and then deionized water was added to the dispersion so as to have a concentration of 0.7%, the mixture was stirred (at 1000 rpm for 5 minutes), and 300 g of a 0.7 mass% aqueous dispersion of the oxidized CNF was obtained. Further, immediately after the concentration adjustment, the Brookfield viscosity was measured by using a B-type viscometer under the conditions of 6 rpm and one minute (viscosity before stirring).

300 g of the aqueous dispersion of oxidized CNF after completion of the measurement of Brookfield viscosity was stirred with Disper for 30 minutes (at 1000 rpm and 23°C). Immediately after the 30-minute stirring, the Brookfield viscosity was measured by using a B-type viscometer under the conditions of 6 rpm and one minute (viscosity after stirring).

<Stability Test 2>

160 g of a 1.0 mass% aqueous dispersion of oxidized cellulose nanofibers was weighed and put in a 600-mL plastic container, and then deionized water was added to the dispersion so as to have a concentration of 0.5%, the mixture was stirred (at 3000 rpm for 10 minutes), and 320 g of a 0.5 mass% aqueous dispersion of the oxidized CNF was obtained. 20 g of the obtained dispersion was left to stand for 6 hours, and then the viscosity at a shear rate of 0.1/s was measured with a rheometer (viscosity before stirring).

The remaining 300 g of the aqueous dispersion of oxidized CNF was transferred to a 500 mL-beaker, and stirred with a stirrer for one day (at 1000 rpm and 23°C). In this regard, the mouth of the beaker was sealed with Parafilm to prevent evaporation of water during the stirring. The aqueous dispersion of oxidized CNF, which had been stirred for one day, was left to stand for 6 hours, and then the viscosity at a shear rate of 0.1/s was measured with a rheometer (viscosity after stirring).

In addition, the viscosity retention for Stability Tests 1 and 2 is calculated by the following equation (4).

$$\text{Equation (4): viscosity retention (\%) = (viscosity after stirring/viscosity before stirring)} \times 100$$

<Example 1>

5.00 g (bone-dry) of bleached unbeaten kraft pulp (brightness: 85%) derived from coniferous trees trees was added to 500 mL of an aqueous solution in which 20 mg of TEMPO (manufactured by Sigma-Aldrich Co. LLC, 0.025 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 2.2 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 93%, the time required for the oxidation reaction was 60 minutes, and the amount of carboxy groups (hereinafter, may also be referred to as "degree of modification") was 0.72 mmol/g. The obtained pulp was adjusted to 1.0% (w/v) with water, and disintegration treatment was performed by using a high-pressure homogenizer until sufficiently high transparency was obtained, and as a result of which an aqueous dispersion of oxidized cellulose nanofibers having a transparency of 93.7% was obtained. The average fiber diameter was 4 nm, and the aspect ratio was 280. Stability Test 1 was carried out on this aqueous dispersion of oxidized CNF, and the values of the Brookfield viscosity before and after stirring were obtained. The viscosity retention at this time was 52.5%.

<Example 2>

5.00 g (bone-dry) of bleached unbeaten kraft pulp (brightness: 85%) derived from coniferous trees was added to 500 mL of an aqueous solution in which 20 mg of TEMPO (manufactured by Sigma-Aldrich Co. LLC, 0.025 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 1.8 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 93%, the time required for the oxidation reaction was 60 minutes, and the amount of carboxy groups was 0.58 mmol/g. The obtained pulp was adjusted to 1.0% (w/v) with water, and disintegration treatment was performed by using a high-pressure homogenizer until sufficiently high transparency was obtained, and as a result of which an aqueous dispersion of oxidized cellulose nanofibers having a transparency of 77.4% was obtained. The average fiber diameter was 4 nm, and the aspect ratio was 320. Stability Test 1 was carried out on this aqueous dispersion of oxidized CNF, and the values of the Brookfield viscosity before and after stirring were obtained. The viscosity retention at this time was 80.3%. Further, Stability Test 2 was carried out on the aqueous dispersion of oxidized CNF, and the values of the viscosity before and after stirring were obtained with a rheometer. The viscosity retention in a low shear rate range at this time was 44.9%.

<Example 3>

5.00 g (bone-dry) of bleached unbeaten kraft pulp (brightness: 85%) derived from coniferous trees was added to 500 mL of an aqueous solution in which 20 mg of TEMPO (manufactured by Sigma-Aldrich Co. LLC, 0.025 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 1.8 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 93%, the time required for the oxidation reaction was 60 minutes, and the amount of carboxy groups was 0.58 mmol/g. The obtained pulp was adjusted to 1.0% (w/v) with water, and disintegration treatment was performed by using a high-pressure homogenizer until sufficiently high transparency was obtained, and as a result of which an aqueous dispersion of oxidized cellulose nanofibers having a transparency of 90.2% was obtained. The average fiber diameter was 4 nm, and the aspect ratio was 350. Stability Test 1 was carried out on this aqueous dispersion of oxidized CNF, and the values of the Brookfield viscosity before and after stirring were obtained. The viscosity retention at this time was 71.4%. Further, Stability Test 2 was carried out on the aqueous dispersion of oxidized CNF, and the values of the viscosity before and after stirring were obtained with a rheometer. The viscosity retention in a low shear rate range at this time was 65.5%.

<Example 4>

5.00 g (bone-dry) of bleached unbeaten kraft pulp (brightness: 85%) derived from coniferous trees was added to 500 mL of an aqueous solution in which 39 mg of TEMPO (manufactured by Sigma-Aldrich Co. LLC, 0.05 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 6.0 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 90%, the time required for the oxidation reaction was 90 minutes, and the amount of carboxy groups was 1.53 mmol/g. The obtained pulp was adjusted to 1.0% (w/v) with water, and disintegration treatment was performed by using a high-pressure homogenizer, so that an aqueous dispersion of oxidized cellulose nanofibers was obtained. The average fiber diameter was 3 nm, and the aspect ratio was 250.
The aqueous dispersion of oxidized CNF was subjected to desalting treatment using a cation exchange resin, and an aqueous dispersion of H-type oxidized CNF was obtained. The proportion of the carboxy groups at this time was 75%. Stability Test 1 was carried out on this aqueous dispersion of H-type oxidized CNF, and the values of the Brookfield viscosity before and after stirring were obtained. The viscosity retention at this time was 99.6%.

<Example 5>

5.00 g (bone-dry) of bleached unbeaten kraft pulp (brightness: 85%) derived from coniferous trees was added to 500 mL of an aqueous solution in which 39 mg of TEMPO (manufactured by Sigma-Aldrich Co. LLC, 0.05 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 6.0 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 90%, the time required for the oxidation reaction was 90 minutes, and the amount of carboxy groups was 1.53 mmol/g. The obtained pulp was adjusted to 1.0% (w/v) with water, and disintegration treatment was performed by using a high-pressure homogenizer, so that an aqueous dispersion of oxidized cellulose nanofibers was obtained. The average fiber diameter was 3 nm, and the aspect ratio was 250.
Stability Test 1 was carried out on the above-prepared 1.0 mass% aqueous dispersion of oxidized cellulose nanofibers. In this regard, in Stability Test 1, when the concentration was adjusted to 0.7% by mass, calcium acetate monohydrate in an amount of 50 mol% was added with respect to the amount of carboxy groups of CNF, and the mixture was stirred at 1000 rpm for 5 minutes. The values of the Brookfield viscosity before and after stirring were obtained for the CNF aqueous dispersion to which the calcium ions had been added. The viscosity retention was 71.6%.

<Comparative Example 1>

5.00 g (bone-dry) of bleached unbeaten kraft pulp (brightness: 85%) derived from coniferous trees was added to 500 mL of an aqueous solution in which 39 mg of TEMPO (manufactured by Sigma-Aldrich Co. LLC, 0.05 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 6.0 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 90%, the time required for the oxidation reaction was 90 minutes, and the amount of carboxy groups was 1.53 mmol/g. The obtained pulp was adjusted to 1.0% (w/v) with water, and disintegration treatment was performed by using a high-pressure homogenizer, so that an aqueous dispersion of oxidized cellulose nanofibers having a transparency of 95.1% was obtained. The average fiber diameter was 3 nm, and the aspect ratio was 250. Stability Test 1 was carried out on this aqueous dispersion of oxidized CNF, and the values of the Brookfield viscosity before and after stirring were obtained. The viscosity retention at this time was 38.2%.

<Comparative Example 2>

5.00 g (bone-dry) of bleached unbeaten kraft pulp (brightness: 85%) derived from coniferous trees was added to 500 mL of an aqueous solution in which 39 mg of TEMPO (manufactured by Sigma-Aldrich Co. LLC, 0.05 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 3.1 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 90%, the time required for the oxidation reaction was 90 minutes, and the amount of carboxy groups was 1.08 mmol/g. The obtained pulp was adjusted to 1.0% (w/v) with water, and disintegration treatment was performed by using a high-pressure homogenizer, so that an aqueous dispersion of oxidized cellulose nanofibers having a transparency of 94.2% was obtained. The average fiber diameter was 3 nm, and the aspect ratio was 260. Stability Test 1 was carried out on this aqueous dispersion of oxidized CNF, and the values of the Brookfield viscosity before and after stirring were obtained. The viscosity retention at this time was 46.7%.

<Comparative Example 3>

5.00 g (bone-dry) of bleached unbeaten kraft pulp (brightness: 85%) derived from coniferous trees was added to 500 mL of an aqueous solution in which 39 mg of TEMPO (manufactured by Sigma-Aldrich Co. LLC, 0.05 mmol per gram of bone-dry cellulose) and 514 mg of sodium bromide (1.0 mmol per gram of bone-dry cellulose) had been dissolved, and the mixture was stirred until the pulp was homogeneously dispersed. A sodium hypochlorite aqueous solution was added to the reaction system so that the sodium hypochlorite was 6.0 mmol/g, and the oxidation reaction was started. The pH in the system decreased during the reaction, but in order to adjust the pH to 10, a 3M aqueous sodium hydroxide solution was sequentially added. The reaction was terminated at the time point when the sodium hypochlorite was consumed and the pH in the system did not change. The mixture after the reaction was filtered through a glass filter to separate pulp, and the pulp was thoroughly washed with water to obtain oxidized pulp (carboxylated cellulose). The pulp yield at this time was 90%, the time required for the oxidation reaction was 90 minutes, and the amount of carboxy groups was 1.53 mmol/g. The obtained pulp was adjusted to 1.0% (w/v) with water, and disintegration treatment was performed by using a high-pressure homogenizer, so that an aqueous dispersion of oxidized cellulose nanofibers was obtained. The average fiber diameter was 3 nm, and the aspect ratio was 250.

Stability Test 1 was carried out on the above-prepared 1.0 mass% aqueous dispersion of oxidized cellulose nanofibers. In this regard, in Stability Test 1, when the concentration was adjusted to 0.7% by mass, calcium acetate monohydrate in an amount of 30 mol% was added with respect to the amount of carboxy groups of CNF, and the mixture was stirred at 1000 rpm for 5 minutes. The values of the Brookfield viscosity before and after stirring were obtained for the CNF aqueous dispersion to which the calcium ions had been added. The viscosity retention was 39.6%.

[Table 1]

| No. | Degree of modification | Transparency (1% concentration) | Counter ion | Ca-ion addition rate | Viscosity retention (Stability Test 1) | Viscosity retention (Stability Test 2) |
|---|---|---|---|---|---|---|
| | mmol/g | % | | mol% | % (6rpm) | % (0.1/s) |
| Example 1 | 0.72 | 93.7 | Na | - | 52.5 | - |
| Example 2 | 0.58 | 77.4 | Na | - | 80.3 | 44.9 |
| Example 3 | 0.58 | 90.2 | Na | - | 71.4 | 65.5 |
| Example 4 | 1.53 | - | H | - | 99.6 | 76.7 |
| Example 5 | 1.53 | - | Na/Ca | 50 | 71.6 | 59.8 |

(continued)

| No. | Degree of modification | Transparency (1% concentration) | Counter ion | Ca-ion addition rate | Viscosity retention (Stability Test 1) | Viscosity retention (Stability Test 2) |
|---|---|---|---|---|---|---|
| | mmol/g | % | | mol% | % (6rpm) | % (0.1/s) |
| Comparative Example 1 | 1.53 | 95.1 | Na | - | 38.2 | 12.3 |
| Comparative Example 2 | 1.08 | 94.2 | Na | - | 46.7 | - |
| Comparative Example 3 | 1.53 | - | Na/Ca | 30 | 39.6 | 4.3 |

As can be understood from Table 1, the oxidized cellulose nanofiber according to the present invention is excellent in the viscosity retention even in a case of being stirred for 30 minutes with Disper at a rotation speed of 1,000 rpm.

**Claims**

1. An oxidized cellulose nanofiber, satisfying a following condition:
   an aqueous dispersion of the oxidized cellulose nanofiber which has undergone concentration adjustment has a retention of a viscosity of 50% or higher measured immediately after 30-minute stirring with Disper at a rotation speed of 1,000 rpm, with respect to the viscosity of the aqueous dispersion of the oxidized cellulose nanofiber measured immediately after the concentration adjustment.

2. The oxidized cellulose nanofiber according to claim 1, satisfying a following condition:
   a 0.7 mass% adjusted aqueous dispersion of the oxidized cellulose nanofiber which has undergone concentration adjustment has a retention of a Brookfield viscosity (at a rotation speed of 6 rpm) of 50% or higher measured immediately after 30-minute stirring with Disper at a rotation speed of 1,000 rpm, with respect to the Brookfield viscosity (at a rotation speed of 6 rpm) of the 0.7 mass% adjusted aqueous dispersion of the oxidized cellulose nanofiber measured immediately after the concentration adjustment.

3. The oxidized cellulose nanofiber according to claim 1 or 2, wherein an amount of carboxy groups of the oxidized cellulose nanofiber is 0.4 to 1.0 mmol/g with respect to a bone dry mass of the oxidized cellulose nanofiber.

4. The oxidized cellulose nanofiber according to claim 3, wherein transparency in a 1.0 mass% aqueous dispersion of the oxidized cellulose nanofiber is 80% or more.

5. The oxidized cellulose nanofiber according to claim 1 or 2, wherein an amount of carboxy groups of the oxidized cellulose nanofiber is 0.2 to 2.0 mmol/g with respect to a bone dry mass of the oxidized cellulose nanofiber, and further a proportion of the carboxy groups in the oxidized cellulose nanofiber, determined by the following equation (1):

$$\text{Proportion of carboxy groups (\%)} = (\text{amount of carboxy groups/total amount of carboxy groups and carboxylate groups}) \times 100,$$

is 50% or more.

6. The oxidized cellulose nanofiber according to claim 1 or 2, wherein an amount of carboxy groups of the oxidized cellulose nanofiber is 0.2 to 2.0 mmol/g with respect to a bone dry mass of the oxidized cellulose nanofiber, and a polyvalent metal is contained in an amount of 40 to 100 mol% with respect to the amount of the carboxy groups of the oxidized cellulose nanofiber.

7. A dispersion of oxidized cellulose nanofiber, comprising the oxidized cellulose nanofiber according to any one of claims 1 to 6.

## INTERNATIONAL SEARCH REPORT

| International application No. |
| --- |
| PCT/JP2019/032536 |

### A. CLASSIFICATION OF SUBJECT MATTER
Int.Cl. C08B15/04(2006.01)1, A23L29/262(2016.01)n, A61K8/73(2006.01)n, D21H11/20(2006.01)n

According to International Patent Classification (IPC) or to both national classification and IPC

### B. FIELDS SEARCHED
Minimum documentation searched (classification system followed by classification symbols)
Int.Cl. C08B15/04, A23L29/262, A61K8/73, D21H11/20

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)
CAplus/REGISTRY(STN), JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | 磯貝明, TEMPO 酸化セルロースナノファイバーの調製と特性解析, 東京大学農学部演習林報告, vol. 126, February 2012, pp. 1-43, (ISOGAI, Akira, Preparation and characterization of TEMPO-oxidized cellulose nanofibers), non-official translation (Bulletin of the University of Tokyo Forests) | 1-4, 6, 7 |
| X | WO 2010/089948 A1 (KAO CORPORATION) 12 August 2010, entire text, examples 1, 2, 4-12, etc. & US 2012/0000392 A1, examples 1, 2, 4-12 & EP 2395027 A1 & CN 102264766 A | 6, 7 |

☒ Further documents are listed in the continuation of Box C.　　☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 10.09.2019 | 01.10.2019 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/032536 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2014-520182 A (UPM-KYMMENE CORPORATION) 21 August 2014, entire text, tables 1, 2, 3, 4, 5, 7, 8, 9, 10, fig. 6, 8, 15, etc. & US 2014/0110070 A1 & WO 2012/168562 A1, tables 1-5, 7-10, fig. 6, 8, 15 & EP 2718329 A1 & CN 103748116 A | 1-4, 7 |
| X | WO 2009/069641 A1 (THE UNIVERSITY OF TOKYO) 04 June 2009, entire text, tables 2, 3, 4, etc. & US 2010/0233481 A1, tables 2-4 & EP 2216345 A1 & CN 101874043 A | 1-4, 7 |
| X | JP 2011-046793 A (THE UNIVERSITY OF TOKYO) 10 March 2011, entire text, table 1, etc. (Family: none) | 1-4, 7 |
| X | JP 2016-528341 A (UPM-KYMMENE CORPORATION) 15 September 2016, entire text, examples 1-4, table 1, etc. & US 2016/0160440 A1 & WO 2015/015056 A1, examples 1-4, table 1 & EP 3027658 A1 & CN 105579474 A | 1-4, 7 |
| X | JP 2016-504445 A (UPM-KYMMENE CORPORATION) 12 February 2016, entire text, tables 2, 3, 4, etc. & US 2015/0322171 A1 & WO 2014/091086 A1, tables 2-4 & EP 2931969 A1 & CN 104854275 A | 1-4, 7 |
| X | JP 2016-529377 A (UPM-KYMMENE CORPORATION) 23 September 2016, entire text, tables 1, 2, 3, 4 etc. & US 2016/0201261 A1 & WO 2015/028719 A1, tables 1-4 & EP 3041871 A1 & CN 105518029 A | 1-4, 7 |
| X | JP 2017-501865 A (UPM-KYMMENE CORPORATION) 19 January 2017, entire text, table 1, etc. & US 2016/0288115 A1 & WO 2015/086901 A1, table 1 & EP 3080082 A1 & CN 105980359 A | 1-4, 7 |
| X | WO 2013/176102 A1 (OJI HOLDINGS CORPORATION) 28 November 2013, entire text, tables 1, 2, etc. & JP 2013-245259 A & JP 2013-253200 A | 1-4, 7 |
| X | WO 2011/089709 A1 (DAI ICHI KOGYO SEIYAKU CO., LTD.) 28 July 2011, entire text, table 1, T1, T2, H1, table 6, S1', S2', H1', etc. & US 2016/0074305 A1 & US 2016/0074306 A1 & EP 2526922 A1 & CN 102724955 A & KR 10-2012-0123371 A | 1-4, 7 |

Form PCT/ISA/210 (continuation of second sheet) (January 2015)

## INTERNATIONAL SEARCH REPORT

International application No.

PCT/JP2019/032536

**Box No. II    Observations where certain claims were found unsearchable (Continuation of item 2 of first sheet)**

This international search report has not been established in respect of certain claims under Article 17 (2)(a) for the following reasons:

1. ☐ Claims Nos.:
   because they relate to subject matter not required to be searched by this Authority, namely:

2. ☒ Claims Nos.: 5
   because they relate to parts of the international application that do not comply with the prescribed requirements to such an extent that no meaningful international search can be carried out, specifically:
   [see extra sheet]

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2019/032536 |

(Continuation of Box No. II)

It is considered that claim 5 of the present invention is an invention defined by the matter below.

"The oxidized cellulose nanofiber according to claim 1 or 2, wherein the amount of the carboxyl group in the oxidized cellulose nanofiber is 0.2-2.0 mmol/g with respect to the absolute dry mass of the oxidized cellulose nanofiber, and the proportion of the carboxyl group in the oxidized cellulose nanofiber obtained by the expression (1) below is 50% or more.

Expression (1): the proportion of the carboxyl group (%) = (the amount of the carboxyl group / a total amount of the amount of the carboxyl group and the amount of the carboxylate group) × 100".

Considering the definition wherein "the proportion of the carboxyl group is 50% or more", it is considered that the invention includes the mode wherein all in the range of "0.2-2.0 mmol/g" are the "carboxyl group".

Meanwhile, the present description, in the comparative examples, discloses oxidized cellulose nanofibers containing the carboxyl group at an amount of 1.53 mmol/g, and at an amount of 1.08 mmol/g, and clarifies that they did not meet a parameter for the present invention.

Considering this, it is considered that the parameter for the amount of the carboxyl group recited in claim 5 includes an invention that does not meet the parameter of a viscosity retaining ratio, which is the problem for the present invention. Thus the claim does not fulfill the requirement for support under PCT Article 6. In addition, it cannot be said that the present description discloses, for the invention of claim 5, a specific method for meeting a parameter for the present invention. Thus the description also does not fulfill the requirement under PCT Article 5.

For the reasons above, for the invention of claim 5, the ISR has not been drawn up.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008001728 A